# EUROPEAN PATENT APPLICATION

(11) **EP 0 938 880 A2**
(43) Date of publication of application: **01.09.1999**
(21) Application number: 99301451.3
(22) Date of filing: 26.02.1999
(51) Int. Cl.: A61F 2/06

(54) **A hand-held stent crimping device**

(30) Priority: 26.02.1998 US 32472
(71) Applicant: Advanced Cardiovascular Systems, Inc., Santa Clara, California 95054 (US)
(72) Inventor: Jackson, Gregg A., Mountain View, California 94041 (US)
(74) Representative: McLeish, Nicholas Alistair Maxwell

(57) **Abstract**

A device for enabling substantially uniform and tight crimping ofan intravascular stent onto a balloon catheter assembly. The stent crimping device includes at least one compressible and resiliently expandable loop portion, that is expandable radially outwardly to enable supporting of the stent and catheter assembly thereon, and is compressible radially inwardly to substantially uniformly and tightly crimp the stent onto the balloon catheter assembly.

## Description

### BACKGROUND OF THE INVENTION

### Field of the Invention

This invention relates to a stent crimping device of the type that will enable the user to crimp a stent onto the distal end of a balloon dilatation catheter assembly, for example of the kind used in a typical percutaneous transluminal coronary angioplasty (PTCA) procedure.

### Description of the Related Art

In a typical PTCA procedure for compressing lesion plaque against the artery wall to dilate the artery lumen, a guiding catheter is introduced percutaneously into the cardiovascular system of a patient through the brachial or femoral arteries and is advanced through the vasculature until the distal end of the guiding catheter is in the ostium. A guide wire and a dilatation catheter having a balloon on the distal end are introduced through the guiding catheter with the guide wire sliding within the dilatation catheter. The guide wire first is advanced out of the guiding catheter into the patient's coronary vasculature, and the dilatation catheter is advanced over the previously advanced guide wire until the dilatation balloon is properly positioned across the lesion. Once in position across the lesion, a flexible, expandable, pre-formed balloon is inflated to a pre-determined size with radiopaque liquid at relatively high pressures to radially compress the atherosclerotic plaque of the lesion against the inside of the artery wall and to thereby dilate the lumen of the artery. The balloon then is deflated to a small profile, so that the dilatation catheter can be withdrawn from the patient's vasculature and blood flow resumed through the dilated artery. While this procedure is typical, it is not the only method used in angioplasty.

In angioplasty procedures of the kind referenced above, a restenosis of the artery may develop over several months, which may require another angioplasty procedure, a surgical bypass operation, or some method ofrepairing or strengthening the area. To reduce the chance of the development of restenosis and to strengthen the area, a physician can implant an intravascular prosthesis for maintaining vascular patency, typically called a stent. A stent is a device used to hold tissue in place in a vessel or to provide support for a vessel to hold the vessel open so that blood can flow freely therethrough. A variety of devices are known in the art for use as stents, including expandable tubular members, in a variety of patterns, that are able to be crimped onto a balloon catheter and expanded after being positioned intraluminally on the balloon catheter, and which retain their expanded form. Typically, the stent is loaded and crimped onto the balloon portion of the catheter, and is advanced to a location inside the artery at the lesion. The stent then is expanded to a larger diameter by the balloon portion of the catheter, to implant the stent in the artery at the lesion.

However, if the stent is not tightly crimped onto the catheter balloon portion, when the catheter is advanced in the patient's vasculature the stent may move or even slide off the catheter balloon portion in the coronary artery prior to expansion, and may block the flow of blood, requiring procedures to remove the stent.

In procedures where the stent is placed over the balloon portion of the catheter, the stent must be crimped onto the balloon portion to prevent the stent from sliding offthe catheter when the catheter is advanced in the patient's vasculature. In the past this crimping often was done by hand, which procedure does not provide optimum results due to the uneven force being applied, resulting in non-uniform crimps. In addition, it was difficult to judge when a uniform and reliable crimp had been applied. Though some tools, such as ordinary pliers, have been used to apply the stent to the catheter, these tools have not been entirely adequate in achieving a uniform crimp. Moreover, an unevenly crimped stent may result in an unevenly expanded stent in the vessel or artery, which is undesirable.

### SUMMARY OF THE INVENTION

This invention is directed to a vascular prosthesis crimping device which enables a stent to be uniformly and tightly crimped onto the balloon portion of a delivery catheter to better secure the stent onto the catheter for delivery of the stent through the patient's vasculature.

The present invention attempts to solve several problems associated with crimping stents onto balloon catheters.

In an exemplary embodiment of the present invention, the stent crimping device includes a compressible and resiliently expandable loop portion (e.g., nylon, nickel-titanium (NiTi), polymide or the material manufactured under the trade name MYLAR by the E.I. duPont deNemours Company of Wilmington, Delaware) in the tip of a hand tool (or a mechanical device such as a pneumatic cylinder) which is connected to the distal end of jaw portions of the hand tool. The loop portion is compressible radially inwardly by the application of compressive force to the hand tool by the user to substantially uniformly and tightly crimp the stent onto the balloon catheter assembly. The loop portion further is expandable upon release by the user of the compressive force applied to the hand tool, to enable the loop portion to resiliently expand for enabling another stent and balloon catheter assembly to be supported therein for another crimping procedure.

The device enables the stent to be crimped substantially uniformly and tightly onto the distal end of a balloon catheter, reducing the risk that the stent may slide off the catheter balloon portion. It further is easy to use in performing the stent crimping procedure. The device also enables the crimping procedure to be performed repeatedly on stent and balloon catheter assemblies with substantially repeatable crimping force applied thereto.

These and other advantages of the invention will become more apparent from the following detailed description thereof when taken in conjunction with the accompanying drawings.

### BRIEF DESCRIPTION OF THE DRAWINGS

FIGURE 1 is a side elevational view of an exemplary embodiment of the present invention, in which the loop portion of the hand tool is in the expanded condition for supporting the stent positioned on the catheter balloon portion.

FIG. 2 is an elevational view of the exemplary embodiment of the present invention in the expanded loop portion condition as shown in FIG. 1.

FIG. 3 is a side elevational view ofthe exemplary embodiment of the present invention in which the loop portion of the hand tool is in the compressed condition for substantially uniformly and tightly crimping the stent onto the catheter balloon portion.

FIG. 4 is an elevational view of the exemplary embodiment of the present invention in the compressed loop portion condition as shown in FIG. 3.

FIG. 5 is an elevational, partly-fragmentary end view of the stent and balloon catheter supporting member, prior to crimping the stent onto the balloon catheter, taken along line 5-5 in FIG. 2.

FIG. 6 is similar to the FIG. 5 view, after crimping the stent onto the balloon catheter.

FIG. 7 is a partial cross-sectional view depicting another embodiment ofthe invention, where a plurality of loops are tightened by a thumbscrew to compress the stent onto the distal end of the catheter.

FIG. 8 is a cross-sectional view taken along lines 8-8 depicting the wire loops of FIG. 7.

### DETAILED DESCRIPTION OF THE PREFERRED EMBODIMENTS

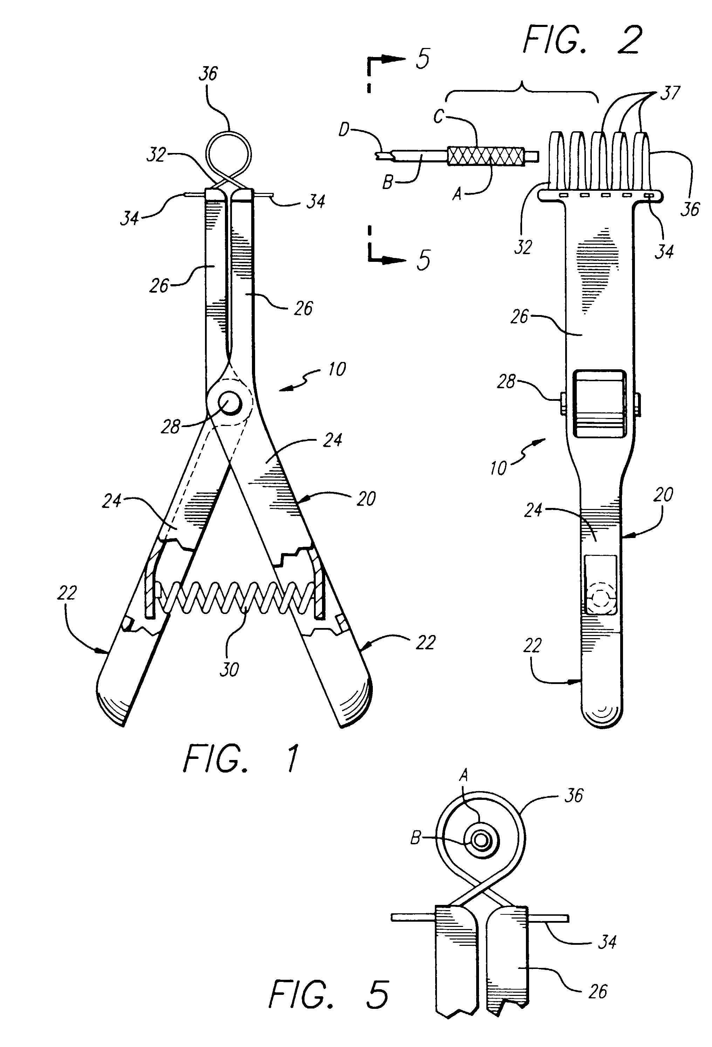

A device 10 comprises a tool 20 for enabling substantially uniform and tight crimping of an intravascular stent A onto the collapsed balloon portion B adjacent to the distal end C of a balloon catheter assembly D. In the exemplary embodiment of the device 10, as shown in FIGS. 1-4, the tool 20 is adapted to be held in the hand of the user, so as to enable the stent A and the catheter D to be supported in the tool 20, and to enable the user to apply compressive force to the tool 20 to substantially uniformly and tightly crimp the stent A onto the catheter D.

The tool 20 includes a pair of arms 22, each arm 22 having handle portions 24 and opposed jaw portions 26. The arms are interconnected pivotally by a pivot pin 28, and the arms normally are held in the expanded condition by a coil spring 30. The spring 30 extends between and biases the handle portions 24 in the normally expanded condition, biasing the jaw portions 26 and the connecting portions 34 in the normally compressed condition and the loop portion 36 in the normally expanded condition. The spring 30 comprises a compressed spring. In the expanded condition of the handle portions 24, the jaw portions 26 are compressed, and in a compressed condition of the handle portions 24, the jaw portions 26 are expanded.

The tool 20 further includes a crimping member 32, connected to the distal ends ofthe jaw portions 26, for supporting the stent A and the the catheter D therein. The crimping member 32 includes portions 34 for connecting the supporting crimping member 32 to the jaw portions 26, and at least one compressible loop portion 36 wherein the portion of the balloon catheter assembly D with the stent A loaded thereon may be supported. The supporting crimping member 32 is comprised of a compressible and resiliently expandable material, such that upon expansion of the jaw portions 26 by compression of the handle portions 24, the jaw end portions 34 are expanded and the loop portion 36 is compressed radially inwardly to substantially uniformly and tightly crimp the stent A onto the catheter D (or onto any stent delivery device). Upon compression of the jaw portions 26, by releasable expansion of the handle portions 24, the distal jaw end portions 34 are drawn together and the loop portion 36 is expanded radially outwardly to support the stent A and the balloon catheter portion B therein.

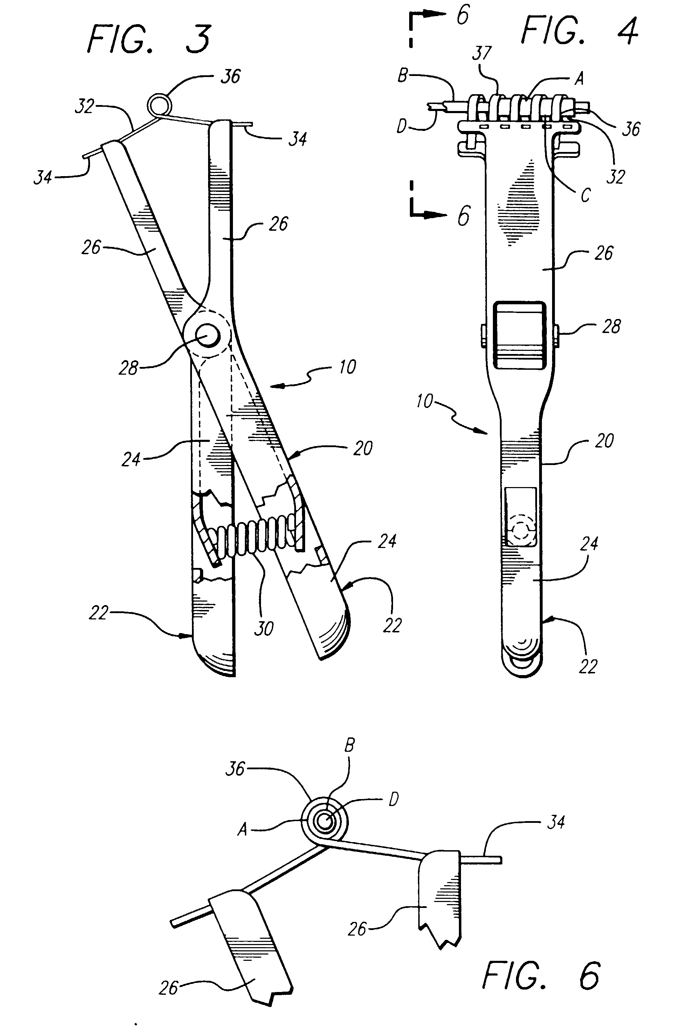

In one embodiment as shown in FIGS. 2 and 4, the loop portion 36 comprises a tubular sleeve including a plurality of discrete spaced-apart interconnected loops 37, wherein the length ofthe sleeve is at least as long as the length of the stent A and the diameter of the sleeve is greater than the diameter of the stent A. Alternatively, the sleeve may comprise a continuous tubular or cylindrical loop portion 36.

The sleeve also may include a slotted mount, to enable tightening or loosening of the sleeve, depending upon the size profiles of the catheter D and the stent A to be crimped thereon.

To enable use of the tool with balloon catheters and stents of various profiles, the crimping member 32 may comprise a threaded-capstan-sleeve configuration, or alternatively, a wire loop or plurality of wire loops, such as a guitar-string-type mechanism for tightening or loosening the sleeve.

In operation, to load the stent A onto the collapsed balloon portion B of the balloon catheter assembly D, the catheter balloon portion B is inserted in the stent A so that the stent A overlies the balloon portion B. To enable the stent A to be crimped onto the catheter balloon portion B or other mechanism for delivering or deploying a stent, the stent A and the catheter balloon portion B may be inserted into and supported in the middle of the loop portion 36 of the crimping member 32. At this point, the stent A is not crimped onto the catheter assembly D, because the stent A has not yet been compressed.

To crimp the stent A onto the catheter balloon portion B, the user of the tool 20 compresses the handle portions 24 together against the biasing force of the compressed spring 30. As the handle portions 24 are compressed, the jaw portions 26 and the connecting portions 34 expand, and the loop portion 36 compresses radially inwardly, compressing the stent A radially inwardly and tightly onto the catheter balloon portion B at a substantially uniform rate.

If further crimping of the stent A onto the catheter balloon portion B is desired, the user may rotate the crimped stent A and the catheter balloon portion B and/or move the two forward or backward in the loop portion 36, and repeat the crimping procedure until the stent A is crimped as tightly onto the catheter balloon portion B as is desired.

After the stent A has been crimped onto the catheter balloon portion B, the user may release the handle portions 24, which return to the expanded condition by reason ofthe biasing force of the compressed spring 30. As the handle portions 24 expand, the jaw portions 26 and the connecting portions 34 compress, and the loop portion 36 expands radially outwardly, permitting the user to remove the catheter balloon portion and the stent, which is now crimped onto the balloon, from the loop portion 36. The balloon catheter assembly D with the stent A crimped thereon then may be inserted into the body of the patient for deployment of the stent A at a desired treatment site.

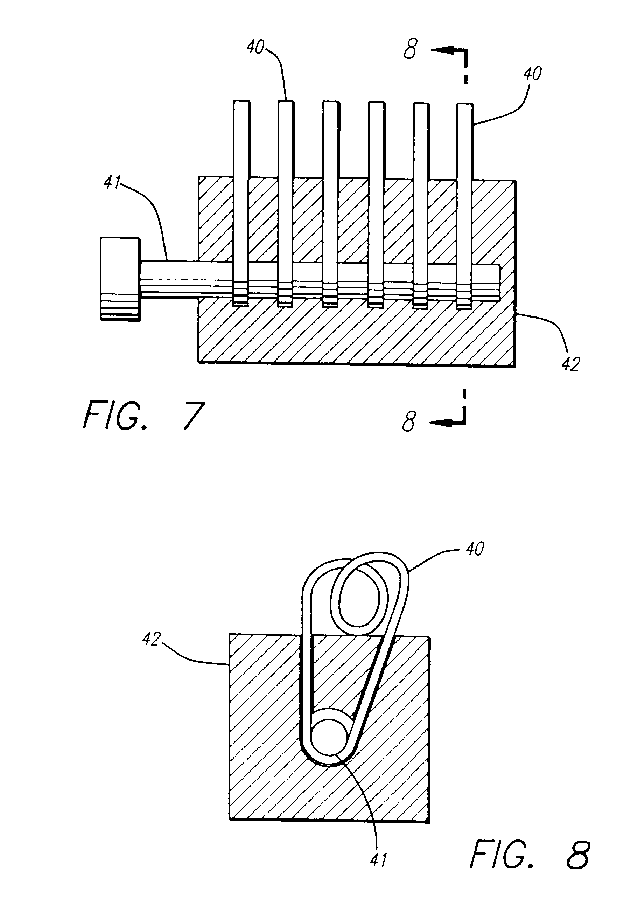

In another embodiment of the invention, as depicted in FIGS. 7 and 8, a plurality of the wire loops 40 are wound around a thumbscrew 41. As the thumbscrew 41 is tightened, for example by hand, the diameter of the wire loops 40 will decrease. The supporting crimping member 42 supports the thumbscrew 41 and provides a basis for the thumbscrew threads to engage and tighten and to draw down the wire loops 40. As the diameter of the wire loops 40 decreases, a stent mounted on the distal end of a catheter (as depicted in the other drawing figures) can be inserted into the loops and then compressed, crimping the stent onto the balloon portion of the catheter. The amount of force applied in crimping the stent is a matter of choice. The embodiment of FIGS. 7 and 8 can be incorporated into the device illustrated in FIGS. 1-6.

While in the preferred embodiment the stent described is intended to be an intraluminal vascular prosthesis for use within a blood vessel, and the balloon delivery catheter is of the same as or is similar to that used in therapeutic coronary angioplasty, it will be appreciated by those skilled in the art that modifications may be made to the present invention to allow the present invention to be used to load any type ofprosthesis onto a delivery device. The present invention is not limited to stents that are deployed in a patient's vasculature, but has wide applications to loading any type of graft, prosthesis, liner or similar structure. Further, the stent may be delivered not only into coronary arteries, but into any body lumen. Other modifications can be made to the present invention by those skilled in the art without departing from the scope thereof.

## Claims

1. A device (10) for crimping a stent (A) onto a balloon catheter (D) or other stent delivery assembly, comprising:
means for supporting (20) a portion (B) of the balloon catheter (D) on which the stent (A) may be loaded, including a compressible portion (32) which is substantially uniformly compressible radially inwardly upon the application of force thereto to substantially uniformly and tightly crimp the stent (B) onto the balloon catheter portion, and is resiliently expandable radially outwardly upon release of the applied force to support the stent (A) and the balloon catheter portion (B) therein; and
means for enabling force (22,26) to be applied to the supporting means (20), adapted to be held in the hand of the user, so as to enable the user to apply compressive force to the force-enabling means (22,26) to generate substantially uniform compression, radially inwardly, of the compressive portion (32) of the supporting means (20), for substantially uniformly and tightly crimping the stent (A) onto the balloon catheter portion (B).

2. A device as in claim 1, further comprising means for biasing (30) the force-applying means (22,26) so as to bias the compressible portion (32) of the supporting means (20) in a normally expanded condition.

3. A device as in claim 1, wherein the compressive portion (32) ofthe supporting means (20) comprises a sleeve including at least one loop (36) for supporting the stent (A) and the balloon catheter portion (B) and enabling substantially uniform and tight crimping of the stent (A) onto the balloon catheter portion (B).

4. A device as in claim 1, wherein the supporting means (20) comprises a wire, and the compressive portion (32) comprises at least one loop (36) for supporting the stent (A) and the balloon catheter portion (B) and enabling substantially uniform and tight crimping of the stent (A) onto the balloon catheter portion (B).

5. A device (10) as in claim 1, wherein the force-application-enabling means (22,26) comprises a pair of pivotally connected arms (22), each including a handle portion (24) and an opposed jaw portion (26), means for pivotally connecting (28) the arms (22) together such that upon expanding the handle portions (24) the jaw portions (26) compress, and upon releasing compression ofthe handle portions (24) the jaw portions (26) expand, and means for connecting (34) the supporting means (20) to the jaw portions (26) such that upon releasing compression of the jaw portions (26) the compressible portion (32) of the supporting means (20) compresses, and upon compressing the jaw portions (26) the compressible portion (32) of the supporting means (20) expands.

6. A device as in claim 2, wherein the biasing means (30) comprises a compressed spring.

7. A device as in claim 3, further comprising means for enabling adjustment of the size (41) of the sleeve dependent upon the size of the stent (A) and the balloon catheter portion (B) to be supported therein.

8. A device as in claim 3, wherein the sleeve comprises a threaded-capstan-type configuration.

9. A device as in claim 4, wherein the wire comprises a guitar-string-type configuration.

10. A device as in claim 5, wherein the supporting means (20) further comprises portions for connecting the supporting means (20) to the connecting means (34).

11. A device as in claim 5, further comprising means for biasing (30) the handle portions (24) in a normally expanded condition.

12. A device as in claim 11, wherein the biasing means (30) comprises a compressed spring.

13. A device as in claim 1, wherein the means for supporting (20) a portion (B) of the balloon catheter assembly (D) on which the stent (A) may be loaded includes a support member (42), and wherein the means for enabling force to be applied to the supporting means (20) includes a thumbscrew (41) which is supported by the support member (42).

14. A device as in claim 13, wherein a plurality of wire loops (37) are attached to the thumbscrew (41) and wherein the diameter of the wire loops (37) is reduced by the application of force to the thumbscrew (41) which thereby crimps the stent (A) onto the balloon catheter portion(B).

15. A method of substantially uniformly and tightly crimping an intravascular stent (A) onto a balloon catheter assembly (D), comprising:
placing a portion (B) ofthe balloon catheter assembly (D), onto which the stent (A) has been positioned, into a radially compressible device (10);
applying a compressive force to compress the radially compressible device (10) radially inwardly, to substantially uniformly and tightly crimp the stent (A) onto the balloon catheter portion (B); and
releasing the compressive force, to enable radially outward expansion of the radially compressible device (10), so that the stent (A) and the balloon catheter portion (B) can be removed therefrom.

16. A method as in claim 15, wherein the step of placing the stent (A) and the balloon catheter portion (B) in a radially compressible device (10) comprises placing the balloon catheter portion (10) in a compressible portion (32) of a means for supporting (20) a stent (A) and a balloon catheter portion (B), in a device that is adapted to be held in the hand of the user, so as to enable the user to apply compressive force to the stent (A) positioned on the balloon catheter portion (B).

17. A method as in claim 15, further comprising the step of repeatedly positioning stents (A) and balloon catheter portions (B) into the radially compressible device (10).

18. A method as in claim 15, further comprising biasing the radially compressible device (10) in a normally expanded condition.

19. A method as in claim 15, wherein the step of supporting the stent (A) and the balloon catheter portion (B) in the radially compressible device (10) comprises supporting the stent (A) and the balloon catheter portion in at least one loop (36) in a sleeve, and the step of applying force to the radially compressible device comprises applying force to at least one loop (36) in the sleeve.

20. A method as in claim 15, wherein the step of supporting the stent (A) and the balloon catheter portion (B) in the radially compressible device (10) comprises supporting the stent (A) and the balloon catheter portion (B) in at least one loop (36) in a wire, and the step of applying force to the radially compressible device (10) comprises applying force to at least one loop (36) in the wire.

21. A method as in claim 15, wherein the step of applying compressive force to the radially compressible device (10) comprises applying force to a pair of handle portions (24) pivotally connected (28) to a pair of jaw portions (26) to which are connected the supporting means (20) for the stent (A) and the balloon catheter (B), thereby compressing the handle portions (24) so as to expand the jaw portions (26) and to substantially uniformly compress the supporting means (20), to substantially uniformly and tightly crimp the stent (A) onto the balloon catheter assembly (D).

22. A method as in claim 18, wherein the step of biasing the supporting means (20) comprises normally expanding the radially compressible device (10) by a compressed spring (30).

23. A method as in claim 21, further comprising the step of biasing the handle portions (24) in normally expanded condition.

24. A method as in claim 23, wherein the step of biasing the handle portions (24) comprises maintaining the handle portions (24) in a normally expanded condition with a compressed spring (30).

25. An intravascular stent (A) substantially uniformly and tightly crimped onto a balloon catheter or other stent delivery assembly portion, comprising:
a balloon catheter assembly portion (B); and
a stent (A) substantially uniformly and tightly crimped onto the balloon catheter portion (B) by substantially uniform compressive force applied thereto through a device (10) that is adapted to be held in the hand of the user.

26. A device (10) for crimping a stent (A) onto the expandable portion (B) of a catheter (D), comprising:
a pair of handles (24) pivotally connected (28) to a pair of opposed jaws (26);
a wire having a least one loop (36) and disposed between and attached to the opposed jaws (26);
a spring (30) disposed between the pair of handles (24) for biasing the handles (24) apart, whereby application of force to close the handles (24) causes the opposed jaws (26) to pivotally move apart so that the at least one wire loop (36) becomes smaller in diameter.

27. A device as in claim 26, wherein the wire is configured to include a plurality of wire loops (37) disposed between and attached to the opposed jaws (26).

28. A device as in claim 26, wherein the diameter of the at least one wire loop (36) is adjustable for receiving stents (A) and catheter assemblies (D) of various dimensions.
